# EUROPEAN PATENT SPECIFICATION

(11) **EP 2 148 934 B1**
(45) Date of publication and mention of the grant of the patent: **21.03.2012**
(21) Application number: 08769291.9
(22) Date of filing: 05.05.2008
(51) Int. Cl.: C12Q 3/00, G01N 33/68

(54) **MATRIX MARKER MODEL AND METHODS FOR ASSESSING AND TREATING RHEUMATOID ARTHRITIS**
MATRIX-MARKER-MODELL UND VERFAHREN ZUR ÜBERPRÜFUNG UND BEHANDLUNG VON RHEUMATISCHER ARTHRITIS
MODÈLE DE MARQUEUR DE MATRICE ET PROCÉDÉS POUR ÉVALUER ET TRAITER L'ARTHRITE RHUMATOIDE

(30) Priority: 03.05.2007 US 915724 P
(43) Date of publication of application: 03.02.2010
(73) Proprietor: Janssen Biotech, Inc., Horsham, PA 19044 (US)
(72) Inventor: HAN, Chenglong, Malvern, Pennsylvania 19355 (US); NOEL, Wim, 1980 Zemst (BE); VASTESAEGER, Nathan, 3010 Kessel Lo (BE)
(74) Representative: Marshall, Cameron John
(86) International application number: PCT/US2008/062621
(87) International publication number: WO 2008/137828

(56) References cited:
- US-A1- 2006 141 547
- US-B2- 7 184 814
- SALEEM ET AL: "Biomarkers: Strategies to predict outcome of rheumatoid arthritis" DRUG DISCOVERY TODAY: THERAPEUTIC STRATEGIES, ELSEVIER LNKD- DOI:10.1016/J.DDSTR.2006.02.004, vol. 3, no. 1, 1 April 2006 (2006-04-01), pages 11-16, XP005394583 ISSN: 1740-6773
- VAN DER HELM - VAN MIL AHM ET AL: "A Prediction Rule for Disease Outcome in Patients With Recent-Onset Undifferentiated Arthritis" ARTHRITIS & RHEUMATISM, vol. 56, no. 2, 1 February 2007 (2007-02-01), pages 433-440, XP002582914
- JANSEN LMA ET AL: "Predictors of radiographic joint damage in patients with early rheumatoid arthritis" ANNALS OF THE RHEUMATIC DISEASES, vol. 60, 1 October 2001 (2001-10-01), pages 924-927, XP002582915
- VISSER H: "Early diagnosis of rheumatoid arthritis" BAILLIERE'S BEST PRACTICE AND RESEARCH. CLINICAL REUMATOLOGY, BAILLIERE TINDALL, LONDON, GB LNKD- DOI:10.1016/J.BERH.2004.08.005, vol. 19, no. 1, 1 February 2005 (2005-02-01), pages 55-72, XP004672995 ISSN: 1521-6942
- VISSER HENK ET AL: "How to diagnose rheumatoid arthritis early: a prediction model for persistent (erosive) arthritis" ARTHRITIS & RHEUMATISM, JOHN WILEY & SONS, INC, US, vol. 46, no. 2, 1 February 2002 (2002-02-01), pages 357-365, XP002388616 ISSN: 0004-3591
- KEYSTONE ET AL: 'Radiographic, Clinical, and Functional Outcomes of Treatment With Adalimumab (a Human Anti-Tumor Necrosis Factor Monoclonal Antibody) in Patients With Active Rheumatoid Arthritis Receiving Concomitant Methotrexate Therapy' ARTHRITIS & RHEUMATISM vol. 50, 2004, pages 1400 - 1411, XP008125662

## Description

### FIELD OF THE INVENTION

The invention relates to a method of determining the efficacy of the treatment for RA based on two to several characteristics known to correlate with negative outcomes in RA presented in a matrix. The invention also relates to a method of determining the efficacy of the treatment for RA based on preparing matrix table comprising two to several covariates which significantly correlate with radiological progression. The matrix relates this profile of characteristics or covariates to the probability (risk) of negative outcomes under each of the alternative potential treatments. This can be done for a subject, for example, prior to the manifestation of other gross measurements of clinical response.

### BACKGROUND OF THE INVENTION

Physicians are confronted with individual patients and individualized decision making on a daily basis and use treatments to limit the risk of bad outcome of a disease. The benefit of a treatment has to be weighed against the potential harm and cost of it and, with a growing armamentarium of therapies, choosing the right treatment for the right patient is a challenge. As disease behavior is different in every patient, 'adequate treatment' is also different for every patient. Ideally a physician would like to know in advance whether a certain patient needs a treatment and whether a specific treatment will or will not be effective in that specific patient. In practice this comes down to knowing the probability (or risk) of bad outcome of a disease and the probability of successfully treating a certain patient with a certain drug.

Arguably the most broadly used risk model in medicine is the risk chart used in cardiology, which combines gender, age, systolic blood pressure, total cholesterol and smoking status to predict the 10-year risk of death due to cardiovascular disease (www.escardio.org/Prevention). Incorporation of the separate risk factors of cardiovascular disease in the model makes it an ideal tool to estimate the risk of bad outcome (death) in the individual patient. The simplicity of this model is furthermore that it is ideal for patient education, which is broadly used for from primary to tertiary care. However, the model does not inform the user about differential effectiveness of therapy in the prevention of risk.

Immune mediated inflammatory disorders (IMID's) are characterized by progressive irreversible damage. The progression of the disease is different in different patients suffering from it and different therapies are capable to prevent this damage to a different extent. Therefore, models that can predict outcome across the heterogeneous patient populations suffering from such IMID would be of great value. In the rest of this description, we will focus on one IMID disease in specific, rheumatoid arthritis and on one specific measure of outcome in this disease, radiological progression.

Rheumatoid arthritis (RA) is characterized by joint inflammation leading to cartilage destruction and erosion of sub-cartilaginous bone. Recently, decreasing progression of radiographic joint damage and functional disability in the RA population is seen, and seems to be largely explained by more effective use of disease modifying anti-rheumatic drugs. More effective use of DMARD's consists of more widespread and earlier use (Finkh et al. Ann Rheum Dis 2006;65:1192-1197). In a recent meta-analysis of 12 studies comparing early vs. late initiation of DMARD's, the reduction in progression rate due to earlier initiation of DMARDs was significantly different when compared to patients who started late. The effect of starting such drugs early could account for half of the total effect size of the treatment. The authors furthermore observed that patients with more aggressive disease benefited more from earlier treatment (Finkh et al. Arthritis Rheum 2006;56(6):864-872).

Early introduction of more intensive therapy leads to further improvement in both clinical and radiological outcome. One-year results of a study comparing 3 DMARDs and high-dose corticosteroids (CS) vs. sulphasalazine (SSZ) in a DMARD naïve early RA population (COBRA trial), showed that combination therapy was more efficacious with respect to suppressing disease activity and radiological progression (Boers et al. Lancet 1997;350:309-318). The 5-year follow-up report of this study shows that suppression of the rate of radiological progression was sustained, independent of anti-rheumatic therapy given after the 6-month cycle of intensive combination therapy (Landewe Arthritis Rheum 2002;46(2):309-318). The diverging slopes of radiological progression rate when comparing early vs. late treatment and intensive vs. monotherapy indicate that early intensive intervention can alter the course of the disease (Finkh et al. Arthritis Rheum 2006;56(6):864-872, Landewe Arthritis Rheum 2002;46(2):309-318).

In a systematic review of randomized placebo-controlled trials published in 2003, the efficacy of and rank of pharmaceutical interventions in preventing radiological progression in RA was investigated. In 25 studies with a total of 3907 RA patients, infliximab (IFX), cyclosporine, SSZ, leflunomide, methotrexate (MTX), parenteral gold, CS, auranofin and interleukin 1 receptor antagonist were statistically better than placebo in terms of change in erosion scores. All agents were equivalent statistically, with the exception of IFX, which was superior to the last five agents (Jones Rheumatology 2003;42(1):6-13). The efficacy of IFX, in terms of suppression of radiological progression, was first established in patients with established RA who had failed treatment with MTX (Lipsky et al. N Engl J Med 2000;343:1594-1602), and was later confirmed in a large randomized active controlled study in a DMARD naïve early RA population in which time to treatment was equal for DMARD and IFX treated patient groups (StClair et al. Arthritis Rheum 2004;50:3432-3443). Similar results have also been reported with other biological agents that neutralize tumor necrosis factor alpha (antiTNF) (Genovese et al. Arthritis Rheum. 2002 Jun;46(6):1443-50, Breedveld Arthritis Rheum. 2006;54(1):26-37).

Probability plots from these studies provide detailed insight into the differentiated treatment effects on radiological progression of biological therapy when compared to MTX monotherapy. Although summary descriptive statistics show statistically significant results when comparing treatment arms as a whole, these plots clearly show that progression of radiological damage is a problem of a subset of individual patients only (van der Heijde Arthritis Rheum. 2005 Jan;52(1):49-60). As with any disease leading to irreversible damage, the problem of radiological follow up is that once the damage is observed, it means that therapeutic intervention already comes too late. Especially in those RA patients in whom radiological deterioration occurs rapidly, delay in initiation of highly effective therapy could be detrimental. Prediction of radiological progression seems to be crucial and should enable the clinician to prevent it. Sub-analyses of the biological studies have shown the value of baseline characteristics for the identification of patients who will show progression of joint damage (Breedveld et al. 2004;63:149-55, Smolen et al. Arthritis Rheum 2005;52:1020-30, Smolen et al. Arthritis Rheum 2006;54(3):702-710, Landewe Arthritis Rheum. 2002;46(2):347-56, Landewe Arthritis Rheum 2006;54(10):3119-3125). Aside from these studies, many clinical reports describe the usefulness of a broad array of clinical, biological and genetic markers in the prediction of radiological progression. At this time the use of models to predict (fast) x-ray progression in daily clinical practice is limited. A better prediction model of disease outcome is needed to make better treatment decisions in (early) RA (de Vries-Bouwstra J et al. J Rheumatol. 2006;33(9):1747-53).

### SUMMARY OF THE INVENTION

Accordingly, there is a need to identify, characterize and model markers useful in developing methods for diagnosing of treating rheumatoid arthritis (RA).

We invented a model that helps predict outcome in RA and relates it to the therapeutic regimen that can be used. A method is provided for prognostic or diagnostic assessment of rheumatoid arthritis in a subject, as recited in the claims. A method is provided for prognostic or diagnostic assessment of treatment of rheumatoid arthritis in a subject, comprising:
a) preparing a set of characteristics selected from age, numbers of tender joints, numbers of swollen joints, erythrocyte sedimentation rate (ESR), rheumatoid factor (RF) positivity, or serum C-reactive protein (CRP) level, and joint damage which correlate with negative outcomes in RA with the probability of a negative clinical outcome under alternative treatments;
b) performing a statistical comparison of said alternative treatments with the corresponding clinical outcome negative probability; and
c) selecting one or more of said alternative treatments based on the lowest negative clinical outcome probability, wherein the subject is a patient having rheumatoid arthritis and steps (a) through (c) are performed before, during, or after treatment of the patient with a therapy for rheumatoid arthritis.

Such methods can include wherein steps (a) through (c) are performed during treatment of the patient with a therapy for the rheumatoid arthritis and about 1-30 weeks after commencement of treatment.

Such methods can include wherein the treatment is selected from at least one of an antirheumatic, a TNF antagonist, a muscle relaxant, a narcotic, a non-steroid anti-inflammatory drug (NSAID), an analgesic, and an anesthetic.

Such methods can include wherein said antirheumatic is at least one selected from methotrexate, auranofin, aurothioglucose, azathioprine, gold sodium thiomalate, hydroxychloroquine sulfate, leflunomide, or sulfasalzine.

Such methods can include wherein said TNF antagonist is selected from a small molecule or a biologic that inhibits at least one TNF biological activity.

Such methods can include wherein said TNF antagonist biologic is an TNF-antibody or TNF receptor fusion protein.

Such methods can include wherein wherein said TNFantagonist biologic is selected from infliximab, adalimumab, enteracept or golimumab.

There is described herein a kit for diagnosing RA and/or related diseases or disorders by identifying and using candidate agents and/or targets which modulate such diseases or disorders and for determining the efficacy of the treatment for RA and/or related diseases or disorders based on two or more characteristics known to correlate with negative outcomes in RA with the probability of a negative clinical outcome under each of the alternative treatments.

In one aspect, the RA-related antagonist is an antibody that specifically binds RA-related product. A particular advantage of such antibodies is that they are capable of binding RA-related product in a manner that prevents its action. The method of the present invention thus employs antibodies having the desirable neutralizing property which makes them ideally suited for therapeutic and preventative treatment of disease states associated with various RA-related disorders in human or nonhuman patients. Accordingly, the present disclosure describes a method of treating RA or a related disease or condition in a patient in need of such treatment which comprises administering to the patient an amount of a neutralizing RA-related product antibody to inhibit the RA-related disease or condition.

In another aspect, there is described herein methods for modulating activity of a member of a RA-related comprising contacting a cell with an agent (e.g., antagonist or agonist) that modulates (inhibits or enhances) the activity or expression of the member of the RA-related gene panel such that activity or expression in the cell is modulated. In a preferred aspect, the agent is an antibody that specifically binds to the RA-related gene panel. In other aspects, the modulator is a peptide, peptidomimetic, or other small molecule.

Herein described are methods of treating a subject having RA or related disorder wherein the disorder can be ameliorated by modulating the amount or activity of the RA-related gene panel. The present disclosure also describes methods of treating a subject having a disorder characterized by aberrant activity of the RA-related product or one of their encoding polynucleotide by administering to the subject an agent that is a modulator of the activity of the RA-related product or or a modulator of the expression of a RA-related gene panel.

In one aspect, the modulator is a polypeptide or small molecule compound. In another aspect, the modulator is a polynucleotide. In a particular aspect, the RA-related antagonist is an siRNA molecule, an shRNA molecule, an antisense molecule, a ribozyme, or a DNAzyme capable of preventing the production of RA-related by cells.

The present invention further provides any invention described herein.

### Detailed Description

The invention relates to a method of determining the efficacy of the treatment for RA based on a set of characteristics known to correlate with negative outcomes in RA presented in a matrix. There is herein also described a method of determining the efficacy of the treatment for RA based on preparing matrix table comprising two to several covariates which significantly correlate with radiological progression. The matrix relates this profile of characteristics or covariates to the probability (risk) of negative outcomes under each of the alternative potential treatments. This can be done for a subject, for example, prior to the manifestation of other gross measurements of clinical response.

A method is described for prognostic or diagnostic assessment of treatment of at least one an rheumatoid arthritis related disorder in a subject, comprising:
d) preparing a matrix table comprising set of two or more covariates which significantly correlate with radiological progression in RA with the probability of a negative clinical outcome under each of the alternative treatments;
e) performing a statistical comparison of said alternative treatments with the corresponding clinical outcome negative probability; and
f) selecting one or more of said alternative treatments based on the lowest negative clinical outcome probability. The model consists of a matrix table that combines a number of characteristics known to correlate with negative outcomes in RA. The matrix table relates this profile of characteristics to the probability (risk) of bad outcome under each of the treatment possibilities. As an application of the model we analyzed its use for prediction of the risk of rapid radiological progression of rheumatoid arthritis treated with either MTX monotherapy or MTX + TNF antagonist.

The model matrix combines several factors in easily readable fashion and predicts outcome relative to the treatment regimen a physician can choose to treat an individual patient with. The model is a tool for treatment guidance and patient education.

The present invention thus relates to a method of diagnosing and/or treating RA and/or related diseases or disorders by identifying and using a set of characteristics known to correlate with negative outcomes in RA and determining the treatment that minimizes the probability of negative outcomes. Herein described is a method of determining the efficacy of the treatment for RA based on a set of characteristics known to correlate with negative outcomes in RA presented in a matrix table. The matrix table relates this profile of characteristics to the probability (risk) of bad outcome under each of the treatment possibilities. This can be done for a subject, for example prior to the manifestation of other gross measurements of clinical response. In one aspect, the method of modeling a matrix of two to several characteristics known to correlate with negative outcomes in RA.

In one aspect, a method is described for for prognostic or diagnostic assessment of treatment of at least one an rheumatoid arthritis related disorder in a subject, comprising:
(a) preparing a set of two or more covariates which significantly correlate with radiological progression in RA with the probability of a negative clinical outcome under each of the alternative treatments;
(b) performing a statistical comparison of said alternative treatments with the corresponding clinical outcome negative probability; and
(c) selecting one or more of said alternative treatments based on the lowest negative clinical outcome probability.

In another aspect, the RA-related characteristic profile and treatment profile is provided in a matrix for prognostic or diagnostic purposes, the method comprising:
(a) preparing a set of two or more characteristics known to correlate with negative outcomes in RA with the probability of a negative clinical outcome under each of the alternative treatments;
(b) performing a statistical comparison of said alternative treatments with the corresponding clinical outcome negative probability; and
(c) selecting one or more of said alternative treatments based on the lowest negative clinical outcome probability.

Such methods can include wherein the subject is a patient having, or having the potential to develop, rheumatoid arthritis and steps (a) through (c) are performed before, during, or after treatment of the patient with a therapy for rheumatoid arthritis.

Such methods can include wherein steps (a) through (c) are performed during treatment of the patient with a therapy for the rheumatoid arthritis and about 1-30 weeks after commencement of treatment.

Such methods can include wherein said covariate is selected from at least one of age, numbers of tender joints, numbers of swollen joints, erythrocyte sedimentation rate (ESR), rheumatoid factor (RF) positivity, or serum C-reactive protein (CRP) level, and joint damage.

Such methods can include wherein the treatment is selected from at least one of an antirheumatic, a TNF antagonist, a muscle relaxant, a narcotic, a non-steroid anti-inflammatory drug (NSAID), an analgesic, and an anesthetic.

Such methods can include wherein said antirheumatic is at least one selected from methotrexate, auranofin, aurothioglucose, azathioprine, gold sodium thiomalate, hydroxychloroquine sulfate, leflunomide, or sulfasalzine.

Such methods can include wherein said TNF antagonist is selected from a small molecule or a biologic that inhibits at least one TNF biological activity.

Such methods can include wherein said TNF antagonist biologic is an TNF-antibody or TNF receptor fusion protein.

Such methods can include wherein wherein said TNF antagonist biologic is selected from infliximab, adalimumab, enteracept or golimumab.

There is described herein a kit for diagnosing RA and/or related diseases or disorders by identifying and using candidate agents and/or targets which modulate such diseases or disorders and for determining the efficacy of the treatment for RA and/or related diseases or disorders based on two or more characteristics known to correlate with negative outcomes in RA with the probability of a negative clinical outcome under each of the alternative treatments.

There is described herein agonists and/or antagonists ofthe transcription of the genes or of the gene products of the RA-related and a method of using RA-related antagonists, including antibodies directed toward RA-related products, to treat RA or related disorders.

In one aspect, the RA-related antagonist is an antibody that specifically binds RA-related product. A particular advantage of such antibodies is that they are capable of binding RA-related product in a manner that prevents its action. The method of the present invention thus employs antibodies having the desirable neutralizing property which makes them ideally suited for therapeutic and preventative treatment of disease states associated with various RA-related disorders in human or nonhuman patients. There is described herein a method of treating RA or a related disease or condition in a patient in need of such treatment which comprises administering to the patient an amount of a neutralizing RA-related antibody to inhibit the RA-related disease or condition.

There are also described herein methods for modulating activity of a member of a RA-related comprising contacting a cell with an agent (e.g., antagonist or agonist) that modulates (inhibits or enhances) the activity or expression of the member of the RA-retated gene panel such that activity or expression in the cell is modulated. In a preferred aspect, the agent is an antibody that specifically binds to the RA-related gene panel. In other aspect, the modulator is a peptide, peptidomimetic, or other small molecule.

### Definitions

The following definitions are set forth to illustrate and define the meaning and scope of various terms used to describe the invention herein.

An "activity," a biological activity, and a functional activity of a polypeptide refers to an activity exerted by a gene of the RA-related in response to its specific interaction with another protein or molecule as determined in vivo, in situ, or in vitro, according to standard techniques. Such activities can be a direct activity, such as an association with or an enzymatic activity on a second protein, or an indirect activity, such as a cellular process mediated by interaction of the protein with a second protein or a series of interactions as in intracellular signaling or the coagulation cascade.

An "antibody" includes any polypeptide or peptide containing molecule that comprises at least a portion of an immunoglobulin molecule, such as but not limited to, at least one complementarity determining region (CDR) of a heavy or light chain or a ligand binding portion thereof, a heavy chain or light chain variable region, a heavy chain or light chain constant region, a framework region, or any portion, fragment or variant thereof. The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. For example, antibody fragments include, but are not limited to, Fab (e.g., by papain digestion), Fab' (e.g., by pepsin digestion and partial reduction) and F(ab')2 (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, and single domain antibodies (e.g., V_{H} or V_{L}), are encompassed by the invention (see, e.g., Colligan, et al., eds., Current Protocols in Immunology, John Wiley & Sons, Inc., NY (1994-2007); Colligan et al., Current Protocols in Polypeptide Science, John Wiley & Sons, NY (1997-2007)).

The phrases "labeled biological molecule" or "labeled with a detectable composition" or "labeled with a detectable moiety" as used herein refer to a biological molecule, e.g., a nucleic acid, comprising a detectable composition, i.e., a label, as described in detail, below. The label can also be another biological molecule, as a nucleic acid, e.g., a nucleic acid in the form of a stem-loop structure as a "molecular beacon," as described below. This includes incorporation of labeled bases (or, bases which can bind to a detectable label) into the nucleic acid by, e.g., nick translation, random primer extension, amplification with degenerate primers, and the like. Any label can be used, e.g., chemiluminescent labels, radiolabels, enzymatic labels and the like. The label can be detectable by any means, e.g., visual, spectroscopic, photochemical, biochemical, immunochemical, physical, chemical and/or chemiluminescent detection. The invention can use arrays comprising immobilized nucleic acids comprising detectable labels.

The term "profile" means a pattern and relates to the magnitude and direction of change of a number of features. The profile may be interpreted stringently, i.e., where the variation in the magnitude and/or number of features within the profile displaying the characteristic is substantially similar to a reference profile or it may be interpreted less stringently, for example, by requiring a trend rather than an absolute match of all or a subset of feature characteristics.

The terms "protein," "polypeptide," and "peptide" include "analogs," or "conservative variants" and "mimetics" or "peptidomimetics" with structures and activity that substantially correspond to the polypeptide from which the variant was derived, as discussed in detail above.

A "polypeptide" is a polymer of amino acid residues joined by peptide bonds, and a peptide generally refers to amino acid polymers of 12 or less residues. Peptide bonds can be produced naturally as directed by the nucleic acid template or synthetically by methods well known in the art.

A "protein" is a macromolecule comprising one or more polypeptide chains. A protein may further comprise substituent groups attached to the side groups of the amino acids not involved in formation of the peptide bonds. Typically, proteins formed by eukaryotic cell expression also contain carbohydrates. Proteins are defined herein in terms of their amino acid sequence or backbone and substituents are not specified, whether known or not.

The term "receptor" denotes a molecule having the ability to affect biological activity, in e.g., a cell, as a result of interaction with a specific ligand or binding partner. Cell membrane bound receptors are characterized by an extracellular ligand-binding domain, one or more membrane spanning or transmembrane domains, and an intracellular effector domain that is typically involved in signal transduction. Ligand binding to cell membrane receptors causes changes in the extracellular domain that are communicated across the cell membrane, direct or indirect interaction with one or more intracellular proteins, and alters cellular properties, such as enzyme activity, cell shape, or gene expression profile. Receptors may also be untethered to the cell surface and may be cytosolic, nuclear, or released from the cell altogether. Non-cell associated receptors are termed soluble receptors or ligands.

Publications refer to any scientific or patent publications, or any other information available in any media format including all recorded, electronic or printed formats. The following references describe well-known techniques used herein: Ausubel, et al., ed., Current Protocols in Molecular Biology, John Wiley & Sons, Inc., NY (1987-2007); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2007); Colligan et al., Current Protocols in Protein Science, John Wiley & Sons, NY (1997-2007).

### Therapeutic agents

### Antagonists

As used herein, the term "antagonists" refer to substances which inhibit or neutralize the biologic activity of RA-related genes or proteins. Such antagonists accomplish this effect in a variety of ways. One class of antagonists will bind to the gene product protein with sufficient affinity and specificity to neutralize the biologic effects of the protein. Included in this class of molecules are antibodies and antibody fragments (such as, for example, F(ab) or F(ab')₂ molecules). Another class of antagonists comprises fragments of the gene product protein, muteins or small organic molecules, i.e., peptidomimetics, that will bind to the cognate binding partners or ligands of the gene product, thereby inhibiting the biologic activity of the specific interaction of the gene product with its cognate ligand or receptor. The RA-related gene antagonist may be of any of these classes as long as it is a substance that inhibits at least one biological activity of the gene product.

Antagonists include antibodies directed to one or more regions of the gene product protein or fragments thereof, antibodies directed to the cognate ligand or receptor, and partial peptides of the gene product or its cognate ligand which inhibit at least one biological activity of the gene product. Another class of antagonists include siRNAs, shRNAs, antisense molecules and DNAzymes targeting the gene sequence as known in the art are disclosed herein.

Suitable antibodies include those that compete for binding to RA-related gene products with monoclonal antibodies that block RA-related gene product activation or prevent RA-related gene product binding to its cognate ligand, or prevent RA-related gene product signalling.

The disclosure also describes chimeric or fusion proteins. As used herein, a "chimeric protein" or "fusion protein" comprises all or part (preferably biologically active) of a RA-related gene product polypeptide operably linked to a heterologous polypeptide (i.e., a polypeptide other than the same RA-related gene product polypeptide). Within the fusion protein, the term "operably linked" is intended to indicate that the RA-related gene product polypeptide and the heterologous polypeptide are fused in-frame to each other. The heterologous polypeptide can be fused to the amino-terminus or the carboxyl-terminus of the RA-related gene product polypeptide. In another aspect a RA-related gene product polypeptide or a domain or active fragment thereof can be fused with a heterologous protein sequence or fragment thereof to form a chimeric protein, where the polypeptides, domains or fragments are not fused end to end but are interposed within the heterologous protein framework.

In yet another aspect, the fusion protein is an immunoglobulin fusion protein in which all or part of a RA-related gene product polypeptide is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a RA-related gene product polypeptide. Inhibition of ligand/receptor interaction can be useful therapeutically, both for treating proliferative and differentiative disorders and for modulating (e.g., promoting or inhibiting) cell survival. A preferred aspect of an immunoglobulin chimeric protein is a C_{H}1 domain-deleted immunoglobulin or "mimetibody" having an active polypeptide fragment interposed within a modified framework region as taught in co-pending application PCT WO/04002417. Moreover, the immunoglobulin fusion proteins of the invention can be used as immunogens to produce antibodies directed against a RA-related gene product polypeptide in a subject, to purify ligands and in screening assays to identify molecules that inhibit the interaction of receptors with ligands.

TNF antagonists suitable for compositions, combination therapy, co-administration, devices and/or methods of the present disclosure (further comprising at least one anti body, specified portion and variant thereof, of the present invention), include, but are not limited to, anti-TNF antibodies, antigen-binding fragments thereof, and receptor molecules which bind specifically to TNF; compounds which prevent and/or inhibit TNF synthesis, TNF release or its action on target cells, such as thalidomide, tenidap, phosphodiesterase inhibitors (e.g, pentoxifylline and rolipram), A2b adenosine receptor agonists and A2b adenosine receptor enhancers; compounds which prevent and/or inhibit TNF receptor signalling, such as mitogen activated protein (MAP) kinase inhibitors; compounds which block and/or inhibit membrane TNF cleavage, such as metalloproteinase inhibitors; compounds which block and/or inhibit TNF activity, such as angiotensin converting enzyme (ACE) inhibitors (e.g., captopril); and compounds which block and/or inhibit TNF production and/or synthesis, such as MAP kinase inhibitors.

As used herein, a "tumor necrosis factor antibody," "TNF antibody," "TNFα antibody," or fragment and the like decreases, blocks, inhibits, abrogates or interferes with TNFα activity *in vitro*, *in situ* and/or preferably *in vivo*. For example, a suitable TNF human antibody of the present disclosure can bind TNFα and includes anti-TNF antibodies, antigen-binding fragments thereof, and specified mutants or domains thereof that bind specifically to TNFα. A suitable TNF antibody or fragment can also decrease block, abrogate, interfere, prevent and/or inhibit TNF RNA, DNA or protein synthesis, TNF release, TNF receptor signaling, membrane TNF cleavage, TNF activity, TNF production and/or synthesis.

Chimeric antibody cA2 consists of the antigen binding variable region of the high-affinity neutralizing mouse anti-human TNFα IgG1 antibody, designated A2, and the constant regions of a human IgG1, kappa immunoglobulin. The human IgG1 Fc region improves allogeneic antibody effector function, increases the circulating serum half-life and decreases the immunogenicity of the antibody. The avidity and epitope specificity of the chimeric antibody cA2 is derived from the variable region of the murine antibody A2. In a particular aspect, a preferred source for nucleic acids encoding the variable region of the murine antibody A2 is the A2 hybridoma cell line.

Chimeric A2 (cA2) neutralizes the cytotoxic effect of both natural and recombinant human TNFα in a dose dependent manner. From binding assays of chimeric antibody cA2 and recombinant human TNFα, the affinity constant of chimeric antibody cA2 was calculated to be 1.04x10¹⁰M⁻¹. Preferred methods for determining monoclonal antibody specificity and affinity by competitive inhibition can be found in Harlow, et a/., antibodies: A Laboratory Manual, Cold Spring Harbor Laboratory Press, Cold Spring Harbor, New York, 1988; Colligan et al., eds., Current Protocols in Immunology, Greene Publishing Assoc. and Wiley Interscience, New York, (1992-2007); Kozbor et al., Immunol. Today, 4:72-79 (1983); Ausubel et al., eds. Current Protocols in Molecular Biology, Wiley Interscience, New York (1987-2007); and Muller, Meth. Enzymol., 92:589-601 (1983).

In a particular aspect, murine monoclonal antibody A2 is produced by a cell line designated c134A. Chimeric antibody cA2 is produced by a cell line designated c168A.

Additional examples of monoclonal anti-TNF antibodies that can be used in the present disclosure are described in the art (see, e.g., U.S. Patent No. 5,231,024; Möller, A. et al., Cytokine 2(3):162-169 (1990); U.S. Application No. 07/943,852 (filed September 11, 1992); Rathjen et al., International Publication No. WO 91/02078 (published February 21, 1991); Rubin et al., EPO Patent Publication No. 0 218 868 (published April 22, 1987); Yone et al., EPO Patent Publication No. 0 288 088 (October 26, 1988); Liang, et al., Biochem. Biophys. Res. Comm. 137:847-854 (1986); Meager, et al., Hybridoma 6:305-311 (1987); Fendly et al., Hybridoma 6:359-369 (1987); Bringman, et al., Hybridoma 6:489-507 (1987); and Hirai, et al., J. Immunol. Meth. 96:57-62 (1987) ).

### TNF Receptor Molecules

Preferred TNF receptor molecules useful in the present disclosure are those that bind TNFα with high affinity (see, e.g., Feldmann et al., International Publication No. WO 92/07076 (published April 30, 1992); Schall et al., Cell 61:361-370 (1990); and Loetscher et al., Cell 61:351-359 (1990)) and optionally possess low immunogenicity. In particular, the 55 kDa (p55 TNF-R) and the 75 kDa (p75 TNF-R) TNF cell surface receptors are useful in the present disclosure. Truncated forms of these receptors, comprising the extracellular domains (ECD) of the receptors or functional portions thereof (see, e.g., Corcoran et al., Eur. J. Biochem. 223:831-840 (1994)), are also useful in the present disclosure. Truncated forms of the TNF receptors, comprising the ECD, have been detected in urine and serum as 30 kDa and 40 kDa TNFα inhibitory binding proteins (Engelmann, H. et al., J. Biol. Chem. 265:1531-1536 (1990)). TNF receptor multimeric molecules and TNF immunoreceptor fusion molecules, and derivatives and fragments or portions thereof, are additional examples of TNF receptor molecules which are useful in the methods and compositions of the present disclosure. The TNF receptor molecules which can be used in the disclosure are characterized by their ability to treat patients for extended periods with good to excellent alleviation of symptoms and low toxicity. Low immunogenicity and/or high affinity, as well as other undefined properties, can contribute to the therapeutic results achieved.

TNF receptor multimeric molecules useful in the present invention comprise all or a functional portion of the ECD of two or more TNF receptors linked via one or more polypeptide linkers or other nonpeptide linkers, such as polyethylene glycol (PEG). The multimeric molecules can further comprise a signal peptide of a secreted protein to direct expression of the multimeric molecule. These multimeric molecules and methods for their production have been described in U.S. Application No. 08/437,533 (filed May 9, 1995).

TNF immunoreceptor fusion molecules useful in the methods and compositions of the present disclosure relate to at least one portion of one or more immunoglobulin molecules and all or a functional portion of one or more TNF receptors. These immunoreceptor fusion molecules can be assembled as monomers, or hetero- or homo-multimers. The immunoreceptor fusion molecules can also be monovalent or multivalent. An example of such a TNF immunoreceptor fusion molecule is TNF receptor/IgG fusion protein. TNF immunoreceptor fusion molecules and methods for their production have been described in the art (Lesslauer et al., Eur. J. Immunol. 21:2883-2886 (1991); Ashkenazi et al., Proc. Natl. Acad. Sci. USA 88:10535-10539 (1991); Peppel et al., J. Exp. Med. 174:1483-1489 (1991); Kolls et al., Proc. Natl. Acad. Sci. USA 91:215-219 (1994); Butler et al., Cytokine 6(6):616-623 (1994); Baker et al., Eur. J. Immunol. 24:2040-2048 (1994); Beutler et al., U.S. Patent No. 5,447,851; and U.S. Application No. 08/442,133 (filed May 16, 1995) ). Methods for producing immunoreceptor fusion molecules can also be found in Capon et al., U.S. Patent No. 5,116,964; Capon et al., U.S. Patent No. 5,225,538; and Capon et al., Nature 337:525-531 (1989).

A functional equivalent, derivative, fragment or region of TNF receptor molecule refers to the portion of the TNF receptor molecule, or the portion of the TNF receptor molecule sequence which encodes TNF receptor molecule, that is of sufficient size and sequences to functionally resemble TNF receptor molecules that can be used in the present disclosure (e.g., bind TNFα with high affinity and possess low immunogenicity). A functional equivalent of TNF receptor molecule also includes modified TNF receptor molecules that functionally resemble TNF receptor molecules that can be used in the present disclosure (e.g., bind TNFα with high affinity and possess low immunogenicity). For example, a functional equivalent of TNF receptor molecule can contain a "SILENT" codon or one or more amino acid substitutions, deletions or additions (e.g., substitution of one acidic amino acid for another acidic amino acid; or substitution of one codon encoding the same or different hydrophobic amino acid for another codon encoding a hydrophobic amino acid). See Ausubel et al., Current Protocols in Molecular Biology, Greene Publishing Assoc. and Wiley-Interscience, New York (1987-2007).

### Compositions and Their Uses

In accordance with the invention, the neutralizing anti-RA-related gene product antagonists, such as monoclonal antibodies, described herein can be used to inhibit RA-related gene product activity. Additionally, such antagonists can be used to inhibit the pathogenesis of RA and -related inflammatory diseases amenable to such treatment, which may include, but are not limited to, rheumatic diseases. The individual to be treated may be any mammal and is preferably a primate, a companion animal which is a mammal and most preferably a human patient. The amount of antagonist administered will vary according to the purpose it is being used for and the method of administration.

The RA-related gene antagonists may be administered by any number of methods that result in an effect in tissue in which pathological activity is desired to be prevented or halted. Further, the anti-RA-related gene product antagonists need not be present locally to impart an effect on the RA-related gene product activity, therefore, they may be administered wherever access to body compartments or fluids containing RA-related gene product is achieved. In the case of inflamed, malignant, or otherwise compromised tissues, these methods may include direct application of a formulation containing the antagonists. Such methods include intravenous administration of a liquid composition, transdermal administration of a liquid or solid formulation, oral, topical administration, or interstitial or inter-operative administration. Adminstration may be affected by the implantation of a device whose primary function may not be as a drug delivery vehicle.

For antibodies, the preferred dosage is about 0.1 mg/kg to 100 mg/kg of body weight (generally about 10 mg/kg to 20 mg/kg). If the antibody is to act in the brain, a dosage of about 50 mg/kg to 100 mg/kg is usually appropriate. Generally, partially human antibodies and fully human antibodies have a longer half-life within the human body than other antibodies. Accordingly, the use of lower dosages and less frequent administration is often possible. Modifications, such as lipidation, can be used to stabilize antibodies and to enhance uptake and tissue penetration (e.g., into the brain). A method for lipidation of antibodies is described by Cruikshank et al. ((1997) J. Acquired Immune Deficiency Syndromes and Human Retrovirology 14:193).

The RA-related gene product antagonist nucleic acid molecules can be inserted into vectors and used as gene therapy vectors. Gene therapy vectors can be delivered to a subject by, for example, intravenous injection, local administration (U.S. Pat. No. 5,328,470), or by stereotactic injection (see, e.g., Chen et al. (1994) Proc. Natl. Acad. Sci. USA 91:3054- 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Methods of Treatment

The present disclosure described both prophylactic and therapeutic methods of treating a subject at risk of (or susceptible to) a disorder or having a disorder associated with aberrant expression or activity of a RA-related gene product polypeptide and/or in which the RA-related gene product polypeptide is involved.

The present disclosure describes a method for modulating or treating at least one RA-related disease or condition, in a cell, tissue, organ, animal, or patient, as known in the art or as described herein, using at least one RA-related gene product antagonist. Compositions of RA-related gene product antagonist may find therapeutic use in the treatment of RA or related conditions. Disorders characterized by aberrant expression or activity of the RA-related gene product polypeptides are further described elsewhere in this disclosure.

### Prophylactic Methods

In one aspect, the disclosure describes a method for at least substantially preventing in a subject, a disease or condition associated with an aberrant expression or activity of a RA-related gene product polypeptide, by administering to the subject an agent that modulates expression or at least one activity of the polypeptide. Subjects at risk for a disease that is caused or contributed to by aberrant expression or activity of a RA-related gene product can be identified by, for example, any or a combination of diagnostic or prognostic assays as described herein. Administration of a prophylactic agent can occur prior to the manifestation of symptoms characteristic of the aberrancy, such that a disease or disorder is prevented or, alternatively, delayed in its progression. Depending on the type of aberrancy, for example, an agonist or antagonist agent can be used for treating the subject. The appropriate agent can be determined based on screening assays described herein.

### Therapeutic Methods

Another aspect of the disclosure pertains to methods of modulating expression or activity of RA-related gene or gene product for therapeutic purposes. The modulatory method of the disclosure involves contacting a cell with an agent that modulates one or more of the activities of the polypeptide. An agent that modulates activity can be an agent as described herein, such as a nucleic acid or a protein, a naturally-occurring cognate ligand of the polypeptide, a peptide, a peptidomimetic, or other small molecule. In one aspect, the agent stimulates one or more of the biological activities of the polypeptide. In another aspect, the agent inhibits one or more of the biological activities of the RA-related gene or gene product polypeptide. Examples of such inhibitory agents include antisense nucleic acid molecules and antibodies and other methods described herein. These modulatory methods can be performed in vitro (e.g., by culturing the cell with the agent) or, alternativelly, in vivo (e.g., by administering the agent to a subject). As such, the present disclosure describes methods of treating an individual afflicted with a disease or disorder characterized by aberrant expression or activity of a RA-related gene product polypeptide. In one aspect, the method involves administering an agent (e.g., an agent identified by a screening assay described herein), or combination of agents that modulate (e.g., up-regulates or down-regulates) expression or activity. Inhibition of activity is desirable in situations in which activity or expression is abnormally high or up-regulated and/or in which decreased activity is likely to have a beneficial effect.

While having described the invention in general terms, the embodiments of the invention will be further disclosed in the following examples which should not be construed as limiting the scope of the claims.

### EXAMPLE 1: Matrix Modeling of Inflammatory-Autoimmune Disease Rheumatorid Arthritis Markers

### Patients and study protocol.

Details on the patient population and study protocol on which we have tested our model have been presented previously (StClair et al. Arthritis Rheum 2004;50:3432-3443). Briefly, patients enrolled in the study had active RA, had not previously received MTX, and had a history of persistent synovitis (>3 months but <3 years from date of diagnosis). In addition, patients had >=10 swollen joints (66 joint count), >=12 tender joints (68 joint count), and >=1 of the following: rheumatoid factor (RF) positivity, radiographic erosions of the hands or feet, or serum C-reactive protein (CRP) level >=2.0 mg/dl.

The institutional review boards at participating sites approved the study protocol. All patients provided written informed consent. Patients were randomized in a 4:5:5 ratio to receive placebo, infliximab 3 mg/kg, or infliximab 6 mg/kg at weeks 0, 2, and 6, and then every 8 weeks through week 46. All patients were treated with oral MTX starting at 7.5 mg/week at week 0; MTX doses were increased by 2.5 mg every 1-2 weeks to reach 15 mg/week by week 4 and 20 mg/week by week 8. MTX was administered to all patients through week 54. Patients also received folic acid >=5 mg/week.
Study evaluations and end points.
Clinical and laboratory analyses.

The American College of Rheumatology core set variables (Felson A&R 1993), including numbers of tender and swollen joints, were documented at each visit. The 28 joint count (Prevoo A&R 1995) was used in the creation of our model as it is more appropriate and more frequently used in daily clinical practice as compared to the 66/68 count. The Westergren method (Koepke 1988) was used to determine erythrocyte sedimentation rate (ESR) at weeks 0, 6, 30, and 54; normal ESR levels were <=20 mm/hour for women and <=15 mm/hour for men. CRP levels (normal <0.8 mg/dl) were measured by nephelometry (Stites 1997) at screening and each visit thereafter. (If possible RF testing should be specified as different IG's tested for have different correlation with radiological progression. Radiographs of the hands and feet were obtained within 4 weeks of receiving the first dose of the study drug, at weeks 30 and 54, and upon use of a protocol-prohibited medication or study termination, as applicable. Joint damage was assessed by change in the modified Sharp/van der Heijde score (SHS) (vander Heijde Lancet 1989) from baseline to week 54.
Statistical analysis and modeling.

The objective of the present analysis was to use baseline variables that are identified as predictors of radiographic progression, in a model that relates them to the probability of rapid radiological progression. The ASPIRE study was not specifically designed for this analysis. We also incorporated the treatment regimen, i.e. MTX monotherapy or MTX + IFX, in the model. This allows the model to guide therapeutic decision making.

Of the 1,049 patients enrolled in the ASPIRE trial, 1,004 were prospectively identified as eligible for inclusion in the efficacy analyses prior to unblinding (StClair A&R 2004). Since the 2 infliximab dosing groups showed a similar change in SHS score from baseline to week 54 (StClair A&R 2004), they were combined in the current analysis for comparison with the MTX-only group. Summary statistics of median, mean, and interquartile ranges are presented for continuous variables. Spearman correlation analyses were performed to evaluate the association between baseline covariates and changes in radiographic scores. Tests for statistical significance were performed using analysis of variance on van der Waerden normal scores. For categorical response parameters, treatment group comparisons were made using the chi-square test. Logistic regression analyses were performed to examine the significance of individual variables in predicting worsening of joint damage at week 54 for both treatment regimens. Since it is our intention to create a model that can be used in clinical practice, only covariates which significantly correlated with radiological progression and which are readily available in daily clinic were selected for the model. In an attempt to simplify the model and in order to allow that patient subgroups described with their baseline characteristics also had fair patient representation in the ASPIRE study, trichotomous representation was chosen in case of linear relationships with radiological progression. Facilitating application in daily clinical practice, clinically meaningful thresholds (e.g. upper limits of normal) were chosen where possible. Dichotomous representation of variables was used in case applicable.

Being the more appropriate way of describing radiological results (vander Heijde A&R 2005), the model was based on the probability of rapid radiological progression rather than on means and standard deviations or means and quartiles. Rapid progression was defined as a progression of at least 5 on the SHS score from baseline to week 54. In the SHS scoring method, a joint is considered to be destroyed if it has 4 joint space narrowing score units or 5 erosion score units (Sharp A&R 1985, Sharp A&R 1991). As such the radiological outcome in our model can be described as 'progression equivalent to the destruction of at least one joint per year'; a definition which is practical when trying to explain risk to patients or third party payers. Although there is dilution of SHS progression over all joints that are radiologically assessed, in an analysis done with the same study results, it was shown that erosion progression or joint space narrowing (JSN) was primarily seen in these joints that had erosion or JSN at baseline (vander Heijde ACR 2005). This means that the joints that are showing destruction at baseline have a higher probability to progress to complete destruction than that previously unaffected joints would start to show destruction.

Statistical analyses were performed using the SAS system (SAS Institute, Cary, NC). All statistical tests were 2-sided and were performed at an alpha level of 0.05.

### Results:

### Patient characteristics.

The median age of the patients participating in this study was 51 years (range 18-76 years). The 1,004 patients included in these analyses had a female gender in 68-71% of subjects, a mean age of 50-51 years old and disease duration of 0.8-0.9 years. 71-73% were RF positive. Disease activity was moderate-to-high in terms of swollen and tender joint counts (66/68 joints counted); 21-22 and 31-33 respectively, CRP and ESR; means range 2.6-3.0 mg/dl and 43-45 mm/hour respectively, pain ratings, global assessments, and Health Assessment Questionnaire scores. The mean and median radiographic score at baseline ranged from 11.2-11.6 and 5.1-5.3 respectively. The study population was well balanced across treatment groups for baseline demographic and clinical characteristics (StClair A&R 2004).
Joint damage progression.

The change in the SHS from baseline to week 54 was significantly less among patients receiving combination therapy of MTX with infliximab at 3 mg/kg and MTX with infliximab at 6 mg/kg than among those receiving MTX alone (mean +/- SD 0.4 +/- 5.8, 0.5 +/- 5.6, and 3.7 +/-9.6, respectively; P <0.001 for each comparison). During this time frame, 39% of patients receiving infliximab plus MTX showed an increase in radiographic score, compared with 61% of patients receiving MTX alone (P < 0.001). Eight percent and 23% respectively showed an increase of their radiographic score of at least 5 SHS units. 17/27 (63%) of patients who had a SHS of 0 at baseline did not progress under MTX, while this was 47/59 (80%) of the combined infliximab+MTX group.
Joint damage progression in relation to baseline characteristics.

In patients receiving MTX only, higher swollen joint count (correlation coefficient: 0.156, p=0.0088), ESR (0.268, p<0.0001) CRP (0.242, p<0.0001) and RF levels (0.127, p=0.0324) at baseline correlated with greater joint damage at week 54. Among patients treated with infliximab plus MTX; baseline SHS was inversely correlated (-0.191, p<0.0001) and age (0.081, p=0.0297) was positively correlated with changes in SHS from baseline to week 54. The association between higher SHS at baseline and less radiographic progression at week 54 was largely due to a decrease in SHS in some patients in the infliximab plus MTX group (Smolen A&R 2004).
Matrix model for prediction of rapid radiological progression.

Swollen joint count, ESR, CRP and RF showed a linear correlation with radiological progression and were entered as trichotomous variables in the model. The threshold values for distribution of the population over the variables were chosen to be clinically relevant and to represent significant proportions of the population at baseline. Although age had a linear relationship with increasing radiological progression it did not show such relationship with progression in the MTX treated population. However, when the variable was entered as dichotomous variable, less than or equal to / more than 65 years old, a significant correlation of age with radiological progression was also observed in the MTX-only treated population. Age and therapy were both entered as dichotomous variables. Baseline SHS score was not introduced in the model, as it is not available in daily clinic and as it is associated with better rather than with worse radiological outcome.

A matrix model was created in which the probability of rapid radiological progression at week 54 is related to the relevant disease characteristics of the individual patients at baseline. A color scheme ranging from blue (lowest risk of rapid progression) to red (highest risk of rapid progression) was applied for better visualization of the risk distribution, see table 1.
Therapeutic guidance using the model.

The relative risk reduction of rapid progression for combination treatment with MTX and infliximab compared with MTX alone ranged from 50% in patients with low disease activity (ESR, CRP & SJC) and low RF-titer at baseline regardless of the age category, to 66%-74% in patients with high disease activity, high RF-titer and age <65 and >=65 respectively. The absolute risk reduction of rapid progression ranged from 1%-3% to 20%-35% for these respective groups. This indicates that the relative benefit of infliximab + MTX combination therapy is largest in those groups who are at the highest risk of rapid radiological progression as determined by SJC, CRP, ESR, RF-titer and age at baseline.

Table 1 shows the differences between MTX and combined IFX+MTX groups in mean van der Waerden (VW) normal scores of the change from baseline in total SHS score at week 54 (vertical bars) and the associated 95% confidence intervals (horizontal bars) for subgroups defined by baseline disease characteristics as represented with thresholds as used in the model.

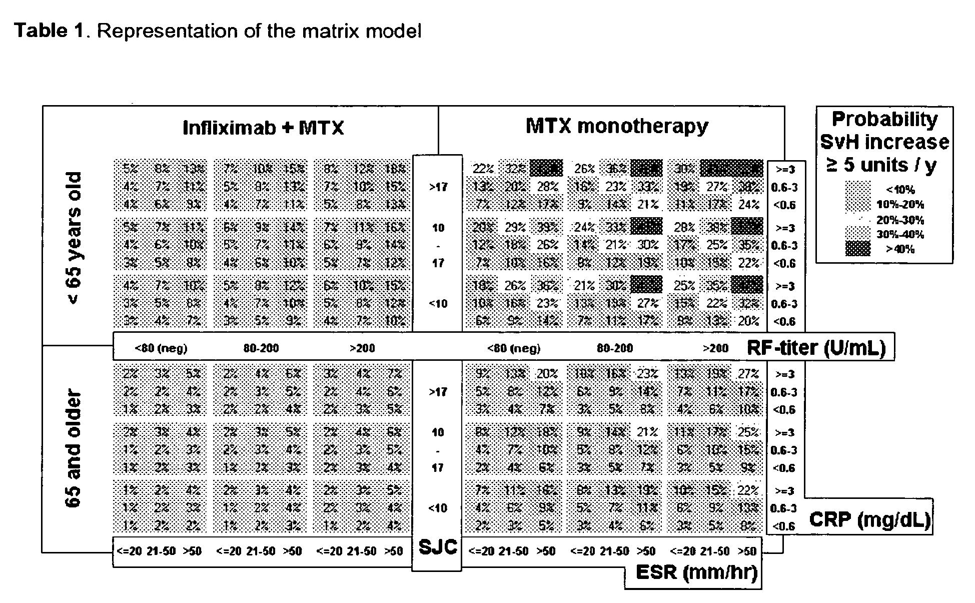

### Advantages

The model is based on what is possibly the most broadly used model for assessing risk in a disease, i.e. the cardiovascular risk chart. The advantage of this model is that it combines several characteristics known to relate to outcome in a way that is easily understandable for patients and physicians. In our model for prediction of outcome in IMID, we use the same way of combining risk factors but also introduce the therapeutic regimen in the risk chart. The advantage of this is that risk expected in the individual is related to the treatment possibilities a physician has. As such, more than just being informative, it can be easily used for therapeutic guidance.

In IMID, patented models that combine disease characterics to predict outcome do exist (e.g. van der Helm-van Mil et al. Arthritis Rheum 2007;56(2):433-440, Drs. van der Helm-van Mil and Huizinga are named as investigators on the patent "Systems and methods for predicting an individual's risk of developing rheumatoid arthritis."). These confirm the high scientific interest for them in these diseases and their patent confirms the need to protect the intellectual property of such models.

The advantage of our Matrix model in comparison to the model mentioned above is that it is more practical as it relates the parameters immediately to the expected risk, without first having to calculate the 'intermediate prediction score' as used by van der Helm-van Mil et al. Also here, incorporation of the therapeutic regimen is an advantage favoring our model.

In conclusion we see three advantages of our model as opposed to the other models we are aware of:
1. we use the combination of characteristics in an easily readable template
2. we provide probability (risk) directly related to the individual patient's profile by providing a matrix reflecting the total heterogeneous population.
3. we include therapeutic regimens into the model which allows the model to be of use for therapeutic guidance.

### References

1. Boers M, Verhoeven AC, Markusse HM, van de Laar MA,Westhovens R, van Denderen JC, et al. Randomised comparison of combined step-down prednisolone, methotrexate and sulphasalazine with sulphasalazine alone in early rheumatoid arthritis. Lancet 1997;350:309-18.
2. St.Clair EW, van der Heijde DM, Smolen JS, Maini RN, Bathon JM, Emery P, et al. Combination of infliximab and methotrexate therapy for early rheumatoid arthritis: a ramdomized, controlled trial. Arthritis Rheum 2004;50:3432-43.
3. Felson DT, Anderson JJ, Boers M, Bombardier C, Chernoff M, Fried B, et al. The American College of Rheumatology preliminary core set of disease activity measures for rheumatoid arthritis clinical trials. Arthritis Rheum 1993;36:729-40.
4. Prevoo ML, van 't Hof MA, Kuper HH, van Leeuwen MA, van de Putte LB, van Riel PL. Modified disease activity scores that include twenty-eight-joint counts: development and validation in a prospective longitudinal study of patients with rheumatoid arthritis. Arthritis Rheum 1995;38:44-8.
5. Landewe RB, Boers M, Verhoeven AC, Westhovens R, Mart AF, van de Laar MA, et al. COBRA combination therapy in patients with early rheumatoid arthritis: long-term structural benefits of a brief intervention. Arthritis Rheum 2002;46:347- 56.
6. Smolen JS, Han C, Bala M, Maini R, Kalden J, van der Heijde D, et al, for the ATTRACT Study Group. Evidence of radiographic benefit of treatment with infliximab plus methotrexate in rheumatoid arthritis patients who had no clinical improvement: a detailed subanalysis from the Anti-Tumor Necrosis Factor Trial in Rheumatoid Arthritis with Concomitant Therapy Study. Arthritis Rheum 2005;52:1020-30.
7. Breedveld FC, Emery P, Keystone E, Patel K, Furst DE, Kalden JR, et al. Infliximab in active early rheumatoid arthritis. Ann Rheum Dis 2004;63:149-55.
8. Genovese MC, Bathon JM, Martin RW, Fleischmann RM, Tesser JR, Schiff MH, Keystone EC, Wasko MC, Moreland LW, Weaver AL, Markenson J, Cannon GW, Spencer-Green G, Finck BK. Etanercept versus methotrexate in patients with early rheumatoid arthritis: two-year radiographic and clinical outcomes. Arthritis Rheum. 2002 Jun;46(6):1443-50.
9. Breedveld FC, Weisman MH, Kavanaugh AF, Cohen SB, Pavelka K, van Vollenhoven R, Sharp J, Perez JL, Spencer-Green GT. The PREMIER study: A multicenter, randomized, double-blind clinical trial of combination therapy with adalimumab plus methotrexate versus methotrexate alone or adalimumab alone in patients with early, aggressive rheumatoid arthritis who had not had previous methotrexate treatment. Arthritis Rheum. 2006 Jan;54(1):26-37.
10. Jones G, Halbert J, Crotty M, Shanahan EM, Batterham M, Ahern M. The effect of treatment on radiological progression in rheumatoid arthritis: a systematic review of randomized placebo-controlled trials. Rheumatology (Oxford). 2003 Jan;42(1):6-13.
11. Landewe RB, Boers M, Verhoeven AC, Westhovens R, van de Laar MA, Markusse HM, van Denderen JC, Westedt ML, Peeters AJ, Dijkmans BA, Jacobs P, Boonen A, van der Heijde DM, van der Linden S. COBRA combination therapy in patients with early rheumatoid arthritis: long-term structural benefits of a brief intervention. Arthritis Rheum. 2002 Feb;46(2):347-56.
12. van der Heijde D, Landewe R, Klareskog L, Rodriguez-Valverde V, Settas L, Pedersen R, Fatenejad S. Presentation and analysis of data on radiographic outcome in clinical trials: experience from the TEMPO study. Arthritis Rheum. 2005 Jan;52(1):49-60.
13. de Vries-Bouwstra J, Le Cessie S, Allaart C, Breedveld F, Huizinga T. Using predicted disease outcome to provide differentiated treatment of early rheumatoid arthritis. J Rheumatol. 2006 Sep;33(9):1747-53. Epub 2006 Jul 15.
14. Annette H. M. van der Helm-van Mil, Saskia le Cessie, Henrike van Dongen, Ferdinand C. Breedveld, Rene' E. M. Toes, and Tom W. J. Huizinga. A Prediction Rule for Disease Outcome in Patients With Recent-Onset Undifferentiated Arthritis. How to Guide Individual Treatment Decisions Arthritis Rheum 2007;56(2):433-440.
15. Saleem B, Cox SR, Emery P. Biomarkers: Strategies to predict outcome of rheumatoid arthritis. Drug Discovery Today: Therapeutic Strategies 2006; 3(1): 11-16.

## Claims

1. A method for prognostic or diagnostic assessment of treatment of rheumatoid arthritis in a subject, comprising:
(a) preparing a set of the characteristics consisting of age, number of tender joints, number of swollen joints, erythrocyte sedimentation rate (ESR), rheumatoid factor (RF) positivity, serum C-reactive protein (CRP) level, and joint damage, which correlate with negative outcomes in RA with the probability of a negative clinical outcome under alternative treatments;
(b) performing a statistical comparison of said alternative treatments with the corresponding clinical outcome negative probability; and
(c) selecting one or more of said alternative treatments based on the lowest negative clinical outcome probability, wherein the subject is a patient having rheumatoid arthritis and steps (a) through (c) are performed before, during, or after treatment of the patient with a therapy for rheumatoid arthritis.

2. The method of claim 1, wherein steps (a) through (c) are performed during treatment of the patient with a therapy for the rheumatoid arthritis and after about 1-30 weeks following commencement of treatment.

3. The method of claim 1, wherein the treatment is selected from an antirheumatic, a TNF antagonist, a muscle relaxant, a narcotic, a nonsteroid anti-inflammatory drug (NSAID), an analgesic, and an anesthetic.

4. The method of claim 3, wherein said antirheumatic is selected from methotrexate, auranofin, aurothioglucose, azathioprine, gold sodium thiomalate, hydroxychloroquine sulfate, leflunomide, and sulfasalzine.

5. The method of claim 3, wherein said TNF antagonist is selected from a small molecule or a biologic that inhibits a TNF biological activity.

6. The method of claim 5, wherein said TNF antagonist biologic is a TNF-antibody or TNF receptor fusion protein.

7. The method of claim 6, where said TNF antagonist biologic is selected from infliximab, adalimumab, enteracept and golimumab.

## Patentansprüche

1. Verfahren zur prognostischen oder diagnostischen Bestimmung der Behandlung von rheumatoider Arthritis in einem Individuum, umfassend:
(a) Erstellen eines Satzes der Eigenschaften, bestehend aus Alter, Anzahl empfindlicher Gelenke, Anzahl geschwollener Gelenke, Erythrocyten-Sedimentationsrate (ESR), Rheumafaktor-(RF)-Positivität, Spiegel des Serum-C-reaktiven Proteins (CRP), und Gelenkbeschädigung, die mit negativen Ergebnissen bei RA mit der Wahrscheinlichkeit eines negativen klinischen Ergebnisses unter alternativen Behandlungen korrelieren;
(b) Durchführen eines statistischen Vergleichs der alternativen Behandlungen mit der entsprechenden Wahrscheinlichkeit für ein negatives klinisches Ergebnis; und
(c) Auswählen von einem oder mehreren der alternativen Behandlungen auf der Basis der niedrigsten Wahrscheinlichkeit für ein negatives klinisches Ergebnis, wobei das Individuum ein Patient mit rheumatoider Arthritis ist, und wobei die Schritte (a) bis (c) vor, während oder nach der Behandlung des Patienten mit einer Therapie für rheumatoide Arthritis durchgeführt werden.

2. Verfahren nach Anspruch 1, wobei die Schritte (a) bis (c) während der Behandlung des Patienten mit einer Therapie für die rheumatoide Arthritis und etwa nach 1 bis 30 Wochen nach Beginn der Behandlung durchgeführt werden.

3. Verfahren nach Anspruch 1, wobei die Behandlung aus einem Antirheumatikum, einem TNF-Antagonisten, einem Muskelrelaxans, einem Narkotikum, einem nichtsteroidalen entzündungshemmenden Arzneimittel (NSAID), einem Analgetikum und einem Anästhetikum ausgewählt ist.

4. Verfahren nach Anspruch 3, wobei das Antirheumatikum aus Methotrexat, Auranofin, Aurothioglucose, Azathioprin, Gold-Natrium-Thiomalat, Hydroxychloroquinsulfat, Leflunomid, und Sulfasalazin ausgewählt ist.

5. Verfahren nach Anspruch 3, wobei der TNFAntagonist aus einem kleinen Molekül oder einem Biologikum ausgewählt ist, das eine biologische TNF-Aktivität hemmt .

6. Verfahren nach Anspruch 6, wobei das TNF-Antagonisten-Biologikum ein TNF-Antikörper oder ein TNF-Rezeptor-Fusionsprotein ist.

7. Verfahren nach Anspruch 6, wobei das TNF-Antagonisten-Biologikum aus Infliximab, Adalimumab, Enteracept und Golimumab ausgewählt ist.

## Revendications

1. Procédé pour l'évaluation diagnostique ou pronostique de traitement de la polyarthrite rhumatoïde chez un sujet, comprenant :
(a) l'élaboration d'un ensemble des caractéristiques consistant en âge, nombre d'articulations sensibles, nombre d'articulations gonflées, vitesse de sédimentation érythrocytaire (VSE), positivité du facteur rhumatoïde (FR), taux de protéine C-réactive (CRP) et atteinte articulaire, qui sont en corrélation avec des issues défavorables dans la PR avec la probabilité d'un résultat clinique négatif sous d'autres traitements possibles ;
(b) l'exécution d'une comparaison statistique desdits autres traitements possibles avec la probabilité négative de résultats cliniques correspondants ; et
c) le choix d'un ou plusieurs desdits autres traitements possible sur la base de la plus faible probabilité de résultats cliniques négatifs, le sujet étant un patient atteint de polyarthrite rhumatoïde et les étapes (a) à (c) étant effectuées avant, pendant ou après traitement du patient par une thérapie pour la polyarthrite rhumatoïde.

2. Procédé selon la revendication 1, dans lequel les étapes (a) à (c) sont effectuées au cours du traitement du patient par une thérapie pour la polyarthrite rhumatoïde et après environ 1-30 semaines après le début du traitement.

3. Procédé selon la revendication 1, dans lequel le traitement est choisi parmi un antirhumatismal, un antagoniste du TNF, un myorelaxant, un narcotique, un anti-inflammatoire non stéroïdien (AINS), un analgésique et un anesthésique.

4. Procédé selon la revendication 3, dans lequel ledit antirhumatismal est choisi parmi le méthotrexate, l'auranofine, l'aurothioglucose, l'azathioprine, l'aurothiomalate sodique, l'hydroxychloroquine sulfate, le léflunomide et la sulfasalazine.

5. Procédé selon la revendication 3, dans lequel ledit antagoniste du TNF est choisi parmi une petite molécule ou une substance biologique qui inhibe l'activité biologique du TNF.

6. Procédé selon la revendication 5, dans lequel ladite substance biologique antagoniste du TNF est un anticorps anti-TNF ou une protéine de fusion de récepteur du TNF.

7. Procédé selon la revendication 6, dans lequel ladite substance biologique antagoniste du TNF est choisie parmi l'infliximab, l'adalimumab, l'enteracept et le golimumab.
